Europäisches Patentamt

European Patent Office

Office européen des brevets

(19)

(11) Veröffentlichungsnummer: **0 153 559**
**B1**

(12) **EUROPÄISCHE PATENTSCHRIFT**

(45) Veröffentlichungstag der Patentschrift:
27.09.89

(21) Anmeldenummer: 85100238.6

(22) Anmeldetag: 11.01.85

(51) Int. Cl.⁴: **A 61 K 6/04, C 22 C 5/04**

(54) Goldarme Dental-Legierungen.

(30) Priorität: 24.02.84 DE 3406711

(43) Veröffentlichungstag der Anmeldung:
04.09.85 Patentblatt 85/36

(45) Bekanntmachung des Hinweises auf die Patenterteilung:
27.09.89 Patentblatt 89/39

(84) Benannte Vertragsstaaten:
AT BE CH DE FR GB IT LI LU NL SE

(56) Entgegenhaltungen:
DE-A- 3 244 802
DE-A- 3 247 399

(73) Patentinhaber: **Degussa Aktiengesellschaft,
Weissfrauenstrasse 9, D-6000 Frankfurt am Main 1 (DE)**

(72) Erfinder: **Rothaut, Josef, Dr. Dipl.-Phys., 247 Harmon
Avenue, Fort Lee NJ 07024 (US)**
Erfinder: **Hausselt, Jürgen, Dr. Dipl.-Phys.,
Weinbergring 37, D-6456 Langenselbold (DE)**
Erfinder: **Steinke, Rudi, Dipl.-Ing., Grünaustrasse 7,
D-6450 Hanau 9 (DE)**
Erfinder: **Klaus, Angela, Feldstrasse 9,
D-6450 Hanau 8 (DE)**

**Beschreibung**

Die Erfindung betrifft goldarme Dental-Legierungen, insbesondere zum Aufbrennen von Dental-Porzellanen, aus 20 bis 35 Gew.% Gold und 45 bis 65 Gew.% Palladium.

In der Zahnheilkunde werden seit langem Kronen und Brücken verwendet, die einen metallischen Kern besitzen und mit einer zahnfarbenen Keramik überzogen sind. Ein solcher Verbund hat gegenüber rein metallischen Kronen und Brücken sowohl ästhetische wie auch physikalische Vorteile, die sich aus der geringen Wärmeleitfähigkeit der Keramik ergeben. Als Legierungen für diese Zwecke haben sich hochgoldhaltige Legierungen bewährt, die ca. 60 bis 90 Gew.% Gold, ca. 1 bis 15 Gew.% Platin, ca. 1 bis 15 Gew.% Palladium, sowie Silber, Zinn, Indium, Gallium, Eisen und kornfeinende Elemente, wie Ruthenium, Iridium oder Rhenium enthalten. Aufgrund der in den vergangenen Jahren drastisch gestiegenen Edelmetallpreise haben sich diese hochgoldhaltigen Legierungen außerordentlich verteuert. Aus diesem Grunde und wegen einer allgemein notwendig gewordenen Kostendämpfung im Gesundheitswesen sind daher in der letzten Zeit eine ganze Reihe von preiswerteren Edelmetall-Legierungen entwickelt worden, die sich für die Verblendung mit einer Dentalkeramik eignen. Es sind dies sowohl goldreduzierte Legierungen auf der Basis Gold-Palladium bzw. Gold-Palladium-Silber mit etwa 45 bis 55 Gew.% Gold als auch Legierungen auf Palladium- und Palladium-Silber-Basis, die kein oder nur wenige Gew.% Gold enthalten.

Legierungen zum Aufbrennen von Dentalporzellanen müssen einer Vielzahl von Anforderungen genügen, wie z.B. gute Gießbarkeit, ausreichende Festigkeit und Duktilität, Verträglichkeit der Elastizitäts- und thermischen Ausdehnungskoeffizienten der Legierung mit denen der handelsüblichen Dental-Keramikmassen, sowie eine ausreichende, jedoch nicht zu hohe Härte. Weiterhin müssen derartige Legierungen bei den Brenntemperaturen der Keramik eine hinreichende Festigkeit besitzen. Außerdem dürfen unerwünschte Reaktionen zwischen Keramik und der Oxidschicht der Legierung, insbesondere Verfärbungen und die Ausbildung von Blasen, nicht auftreten.

Während hochgoldhaltige Legierungen diesen Anforderungen meist genügen, weisen praktisch alle bekannten goldreduzierten und goldfreien Edelmetall-Aufbrennlegierungen einen mehr oder weniger gravierenden Mangel auf. So besitzen die handelsüblichen Legierungen auf der Basis Gold-Palladium mit einem Goldgehalt zwischen 45 und 55 Gew.% fast ausnahmslos ein sehr hohes Schmelzintervall, was zum Teil die Leistungsfähigkeit handelsüblicher Gießapparaturen überfordert. Ein weiterer, den Erfolg einer dentalkeramischen Arbeit gefährdender Schwachpunkt dieses Typs von Legierungen ist ihr durchweg sehr niedriger thermischer Ausdehnungskoeffizient von z.T. weniger als $14,0 \cdot 10^{-6} K^{-1}$, was bei ungünstiger Formgebung der Restauration, bzw. bei nachträglichen Lötarbeiten an verblendeten Metall-Keramik-Teilen zu Sprüngen in der Keramik und auch zu Abplatzungen führen kann. Der gravierendste Nachteil dieser Legierungen liegt jedoch in der Neigung, nach dem Schmelzen im Graphittiegel in der Grenzfläche zwischen Metall und Keramik Blasen zu bilden. Die Hauptursache für diese Blasenbildung liegt darin, daß von der Schmelze aus dem Graphittiegel aufgenommener Kohlenstoff beim Aufbrennen der Keramik mit Oxiden der Legierung oder der Keramik zu Kohlenmonoxid verbrennt, das als Gasblase in der Grenzfläche zurückbleibt (z.B. F. Sperner, dentallabor XXX, Heft 12/1982, S. 1733).

Legierungen auf Palladium-Basis sind ebenfalls mit einer Vielzahl von Unzulänglichkeiten behaftet. Bei diesen Legierungen führt z.B. die Zugabe von Legierungsbestandteilen, die das Schmelzintervall auf notwendige Werte herabsetzen, zu einer unvertretbaren Härtesteigerung. Bei Legierungen auf Palladium-Basis kommt noch hinzu, daß sie aufgrund ihres hohen Palladium-Gehaltes und der damit verbundenen Anfälligkeit gegen Kohlenstoffaufnahme auf keinen Fall in einem Graphittiegel erschmolzen werden dürfen. Die Aufnahme von Kohlenstoff würde die ohnehin bei diesen Legierungen auftretende Sprödigkeit drastisch erhöhen und zudem eine blasenfreie Verblendung mit Dentalkeramik unmöglich machen.

In der DE-OS 2 944 755 werden zahlreiche Aufbrennlegierungen mit ca. 32 bis 63% Gold und 29 bis 58% Palladium beschrieben, die außerdem noch Indium enthalten und denen bis zu einigen Prozenten Gallium, Zinn, Kupfer, Nickel, Aluminium, Titan und Silber zugesetzt sein können. Der Kupfergehalt darf bei diesen Legierungen höchstens 5% betragen. Es hat sich jedoch gezeigt, daß auch solche Legierungen, wenn sie weniger als ca. 40% Gold enthalten, sehr hohe Liquidustemperaturen aufweisen (z.T. über 1400 °C). Eine Reduzierung der Liquidustemperaturen durch Variation der übrigen Zusätze führt zu unvertretbaren Härtewerten.

Es war daher Aufgabe der vorliegenden Erfindung, goldarme Dental-Legierungen zu schaffen, insbesondere zum Aufbrennen von Dentalporzellanen, aus 20 bis 35 Gew% Gold und 45 bis 65 Gew% Palladium, deren Goldgehalt deutlich unter dem der üblichen Gold-Palladium-Sparaufbrennlegierungen liegen soll, die sich leicht verarbeiten lassen, in ihrem thermischen Ausdehnungskoeffizienten den bekannten Dentalporzellanen entsprechen, bei denen weder Verfärbungen der Porzellane noch Blasenbildung nach Schmelzen im Graphittiegel auftreten und die gegenüber bekannten Gold-Sparlegierungen vor allem eine verminderte Härte und ein erniedrigtes Schmelzintervall aufweisen sollen.

Diese Aufgabe wurde erfindungsgemäß dadurch gelöst, daß diese Legierungen zusätzlich enthalten:

a) 6 bis 15 Gew.% Kupfer und 0 bis 10 Gew.% Nickel,

b) 0 bis 12 Gew.% Indium, 0 bis 12 Gew.% Zinn und 0 bis 4 Gew.% Gallium, wobei die Summe der Gehalte von Indium und Zinn addiert mit dem Zweieinhalbfachen des Galliumgehaltes einen Wert zwischen 5 und 15 Gew.% ergeben muß,

c) 0,1 bis 1 Gew.% Iridium und/oder Rhenium und/oder Ruthenium und

d) wahlweise noch 0 bis 1 Gew.% Aluminium, 0 bis 1 Gew.% Tantal, 0 bis 1 Gew.% Titan und/oder 0 bis 5 Gew.% Silber, wobei die Summe aller Komponenten 100% ergibt.

Überraschenderweise hat sich gezeigt, daß die Verwendung von 6 bis 15 Gew.% Kupfer und gegebenenfalls bis zu 10% Nickel die notwendige Senkung des Schmelzintervalls hervorruft und ebenfalls den thermischen Ausdehnungskoeffizienten der Legierung anhebt, ohne jedoch die bei derartigen Zusätzen bereits bei geringen Konzentrationen üblicherweise eintretende drastische Härtesteigerung zu bewirken. Es ist ebenfalls überraschend, daß die bei so hoch kupferhaltigen Legierungen zu erwartende schwarze Oxidfarbe, die sich für die Verblendung mit Keramik als störend erweist, bei den erfindungsgemäßen Legierungen nicht auftritt; es wird vielmehr ein graues und festhaftendes Oxid beobachtet, das gleichzeitig eine außergewöhnlich gute Haftung zwischen dem Metall und der aufgebrannten Dentalkeramik bewirkt. Der Zusatz an Kupfer und Nickel sollte allerdings 16 Gew.% nicht überschreiten.

Als weiterer überraschender Effekt wurde bei kupferhaltigen Legierungen mit mehr als 6 Gew.% Kupfer beobachtet, daß durch Zulegieren von Zinn die Kohlenstoffaufnahme der Schmelze aus dem Graphittiegel reduziert wird. Dieser positive Einfluß von Zinn kann durch die Zugabe von bis zu 1 Gew.% Aluminium und/oder Titan deutlich verstärkt werden. Konzentrationen an Aluminium und Titan über 1 Gew.% führen allerdings zu unvertretbaren Härtesteigerungen der Legierung. Dieser Zusammenhang ermöglicht es, diese Legierungen trotz ihres hohen Palladium-Gehaltes auch nach dem Erschmelzen im Graphittiegel noch problemlos mit Dentalkeramik verblenden zu können, ohne daß durch Kohlenstoff verursachte Blasen in der Grenzfläche zwischen Metall und Keramik auftreten. Diese Möglichkeit, durch legierungstechnische Maßnahmen die Kohlenstoffaufnahme der Schmelze aus einem Graphittiegel drastisch zu reduzieren, ist insofern bemerkenswert, als bisher bekannte Untersuchungen eine mit steigendem Palladium-Gehalt der Legierung zunehmende Neigung festgestellt haben, in flüssigem Zustand Kohlenstoff aufzunehmen. Dieser Zusammenhang führt bei den bekannten goldreduzierten Legierungen häufig zu Blasen in der Keramik, obwohl diese mit 30 bis 40 Gew.% Pd wesentlich Pd-ärmer sind als erfindungsgemäße Legierungen.

Bei Legierungen, die nicht im Graphittiegel erschmolzen werden müssen, ist es auch möglich, den Legierungsbestandteil Zinn ganz oder teilweise durch Indium und/oder Gallium zu ersetzen. Alle Legierungen lassen sich problemlos verarbeiten und zeigen ein außerordentlich gutes Ausfließ- und Formfüllungsvermögen.

Überraschenderweise wurde bei den erfindungsgemäßen Legierungen festgestellt, daß sie trotz ihres hohen Anteils an unedlen Bestandteilen ein außerordentlich gutes Korrosionsverhalten zeigen, das durchaus mit dem hochgoldhaltiger Legierungen vergleichbar ist.

Es ist bekannt, daß der Zusatz von Silber die Verarbeitbarkeit von Edelmetall-Dentallegierungen verbessert, insbesondere deren Gieß- und Lötbarkeit. Die erfindungsgemäßen Legierungen können daher auch 0 bis 5 Gew.% Silber enthalten. Eine Verfärbung der Keramik konnte bei diesen Konzentrationen nicht beobachtet werden.

Als besonders vorteilhaft haben sich Legierungen herausgestellt, insbesondere was deren Härte, Ausdehnungskoeffizient und Schmelzintervall angeht, bei denen die Summe der Gewichtsprozente der Elemente Nickel, Kupfer, Indium, Zinn, Gallium und Gold einen Wert zwischen 23 und 28 ergibt, wobei der Galliumgehalt mit einem Faktor 2,5 und der Goldgehalt mit einem Faktor 0,3 zu multiplizieren sind. Dabei haben sich nickelfreie Legierungen mit 7 bis 12 Gew.% Kupfer besonders bewährt.

In der folgenden Tabelle werden die Eigenschaften einiger Legierungen gemäß der Erfindung angegeben. Der Härtewert «hart» wurde dabei nach einer Glühung von 15 Minuten bei 700 °C gemessen.

## Tabelle

| Nr | Au | Pd | Cu | Ni | In | Sn | Ga | Ti | Al | Ag | Ru | Schmelzintervall $T_{L[°C]}$ $T_S$ | Härte/HV5 Guß | Härte/HV5 hart |
|---|---|---|---|---|---|---|---|---|---|---|---|---|---|---|
| 1 | 25 | 56,8 | 15 | – | – | – | 3 | – | – | – | 0,2 | 1242–1172 | 200 | 216 |
| 2 | 25 | 60,8 | 10 | – | – | 2 | 2 | – | – | – | 0,2 | 1318–1244 | 178 | 180 |
| 3 | 25 | 59,6 | 10 | – | 2 | – | 3 | – | – | – | 0,4 | 1298–1214 | 236 | 240 |
| 4 | 30,5 | 51,7 | 6,3 | – | 10,7 | 0,4 | – | – | – | – | 0,4 | 1244–1313 | 216 | 220 |
| 5 | 30 | 49,6 | 8,2 | – | – | 7,8 | – | – | – | 4 | 0,4 | 1266–1172 | 184 | 190 |
| 6 | 30 | 54,6 | 6,3 | – | 1,9 | 6,8 | – | – | – | – | 0,4 | 1298–1201 | 266 | 300 |
| 7 | 25 | 54 | 6 | 4 | – | 10,2 | – | 0,4 | – | – | 0,4 | 1218–1205 | 250 | 305 |
| 8 | 35 | 47,4 | 8,5 | – | – | 8,5 | – | – | 0,2 | – | 0,4 | 1236–1197 | 278 | 309 |

## Patentansprüche

1. Goldarme Dental-Legierungen, insbesondere zum Aufbrennen von Dentalporzellanen, aus 20 bis 35 Gew.% Gold und 45 bis 65 Gew.% Palladium, dadurch gekennzeichnet, daß sie zusätzlich enthalten:

a) 6 bis 15 Gew.% Kupfer und 0 bis 10 Gew.% Nickel,

b) 0 bis 12 Gew.% Indium, 0 bis 12 Gew.% Zinn und 0 bis 4 Gew.% Gallium, wobei die Summe der Gehalte von Indium und Zinn addiert zum Zweieinhalbfachen des Galliumgehaltes einen Wert zwischen 5 und 15 Gew.% ergeben muß,

c) 0,1 bis 1 Gew.% Iridium und/oder Rhenium und/oder Ruthenium und

d) wahlweise noch 0 bis 1 Gew.% Aluminium, 0 bis 1 Gew.% Tantal, 0 bis 1 Gew.% Titan und/oder 0 bis 5 Gew.% Silber, wobei alle Komponenten eine Summe von 100% ergeben.

2. Dental-Legierungen nach Anspruch 1, dadurch gekennzeichnet, daß die Summe der Gewichtsprozente der Elemente Nickel, Kupfer, Indium, Zinn, Gallium und Gold einen Wert zwischen 23 und 28 ergibt, wobei der Galliumgehalt mit einem Faktor 2,5 und der Goldgehalt mit einem Faktor 0,3 zu multiplizieren sind.

3. Dental-Legierungen nach Anspruch 1 und 2, dadurch gekennzeichnet, daß sie 7 bis 12 Gew.% Kupfer enthalten und nickelfrei sind.

## Claims

1. Low-gold dental alloys, in particular for fusing on dental porcelains, containing 20 to 35% by weight of gold and 45 to 65% by weight of palladium, characterised in that they additionally contain:

a) 6 to 15% by weight of copper and 0 to 10% by weight of nickel,

b) 0 to 12% by weight of indium, 0 to 12% by weight of tin and 0 to 4% by weight of gallium, the sum of the indium and tin contents added to two and a half times the gallium content needing to be equal to a value between 5 and 15% by weight,

c) 0.1 to 1% by weight of iridium and/or rhenium and/or ruthenium and

d) optionally in addition 0 to 1% by weight of aluminium, 0 to 1% by weight of tantalum, 0 to 1% by weight of titanium and/or 0 to 5% by weight of silver, the sum of the components being 100%.

2. Dental alloys according to Claim 1, characterised in that the sum of the percentages by weight of the elements nickel, copper, indium, tin, gallium and gold is a value between 23 and 28, the gallium content needing to be multiplied by a factor of 2.5 and the gold content by a factor of 0.3.

3. Dental alloys according to Claims 1 and 2, characterised in that they contain 7 to 12% by weight of copper and are free of nickel.

## Revendications

1. Alliages dentaires pauvres en or, en particulier pour la calcination de porcelaines dentaires à base de 20 à 35% en poids d'or et 45 à 65% en palladium, caractérisés en ce qu'ils contiennent en outre:

a) de 6 à 15% en poids de cuivre et de 0 à 10% de nickel,

b) de 0 à 12% en poids d'indium, de 0 à 12% en poids d'étain, et de 0 à 4% en poids de gallium pour lesquels la somme des teneurs en indium et en étain additionnée de deux fois et demi de la teneur en gallium doit fournir une valeur comprise entre 5 et 15% en poids,

c) de 0,1 à 1% en iridium et/ou rhénium et/ou ruthénium et,

d) facultativement encore de 0 à 1% en poids d'aluminium, de 0 à 1% en poids de tantale, de 0 à 1% en poids de titane et/ou de 0 à 5% en poids d'argent pour lesquels tous les composants fournissent une somme de 100%.

2. Alliages dentaires selon la revendication 1, caractérisés en ce que la somme des pourcentages pondéraux des éléments nickel, cuivre, indium, étain, gallium, et or fournit une valeur comprise entre 23 et 28, pour laquelle la teneur en gallium doit être multipliée par un facteur de 2,5 et la teneur en or par un facteur de 0,3.

3. Alliages dentaires selon les revendications 1 et 2, caractérisés en ce qu'ils renferment de 7 à 12% en poids de cuivre et sont dépourvus de nickel.